# EUROPEAN PATENT APPLICATION

(11) **EP 4 631 452 A1**
(43) Date of publication of application: **15.10.2025**
(21) Application number: 24177733.3
(22) Date of filing: 23.05.2024
(51) Int. Cl.: A61B 18/14

(54) **SMOKE-ABSORBING ELECTROTOME WITH INTEGRATED TELESCOPIC SMOKE-ABSORBING PIPE AND ELECTRODE PROTECTION SLEEVE THEREOF**

(30) Priority: 12.04.2024 CN 202420758231 U
(71) Applicant: Zhejiang Medstar Technology Co., Ltd., Jiaxing Zhejiang 314006 (CN)
(72) Inventor: WANG, Qinghua, Jiaxing, Zhejiang, 314006 (CN); ZHOU, Genliang, Jiaxing, Zhejiang, 314006 (CN); CHAO, Yu, Jiaxing, Zhejiang, 314006 (CN); LIU, Tianguo, Jiaxing, Zhejiang, 314006 (CN); YIN, Yuhao, Jiaxing, Zhejiang, 314006 (CN)
(74) Representative: Ipey

(57) **Abstract**

The utility model discloses a smoke-absorbing electrotome with an integrated telescopic smoke-absorbing pipe and an electrode protection sleeve thereof, characterized by including an electrotome electrode, a conductive sheet integrated electrode socket, a telescopic pipe, an electrotome handle and a rotating tail joint, the telescopic pipe being formed by integrated injection molding, a hexagonal angle lock being formed in an inner cavity of the telescopic pipe by integrated injection molding, the electrotome electrode and the conductive sheet integrated electrode socket being detachably connected to the hexagonal angle lock respectively, a rotating damping telescopic pipe locking mechanism being arranged between the telescopic pipe and the electrotome handle, and a smoke-discharging corrugated pipe being arranged at a rear end of the rotating tail joint; and also including a multifunctional electrode protection sleeve, the multifunctional electrode protection sleeve being provided with a cleaning end, an electrode protection structure and a pulling end, and the multifunctional electrode protection sleeve being inserted in the telescopic pipe. In the utility model, the telescopic pipe and the electrotome handle are both formed by integrated injection molding, thereby avoiding a falling risk of a split smoke-absorbing pipe. Locking as well as expansion and contraction functions of the telescopic pipe can be achieved by the rotating damping telescopic pipe locking mechanism to adapt to different surgical needs.

## Description

### Technical Field

The utility model relates to the technical field of medical instruments, and in particular to a smoke-absorbing electrotome with an integrated telescopic smoke-absorbing pipe and an electrode protection sleeve thereof.

### Background

An electrotome is a common surgical tool. During surgical cutting, a large amount of smoke is generated, which blocks the line of sight of doctors and thus affects the normal operation of a surgery. Therefore, most of the current electrotomes have a smoke absorption function. The smoke generated during the surgery is absorbed by a vacuum adsorption apparatus to ensure the normal and smooth operation of the surgery.

Smoke-absorbing pipes in existing smoke-absorbing electrotomes are mostly in a split structure, but the smoke-absorbing pipe with the split structure has the falling risk, which increases the risk of the surgery. There is no telescopic locking structure between the existing smoke-absorbing pipe and the existing smoke-absorbing electrotome, and the length of the smoke-absorbing pipe cannot be adjusted flexibly, so that the smoke-absorbing pipe is not applicable to various surgical needs. Furthermore, in clinical practice, when the surgical time is relatively long, there is a situation that blood and body fluids adhere to an electrotome button, which increases the risk of the button being stuck. In addition, in the using process of the existing smoke-absorbing electrotome, the replacement of an electrode and the cleaning of the smoke-absorbing pipe are relatively troublesome. During transportation, the electrode of the smoke-absorbing electrotome cannot be better protected. Therefore, the utility model provides a smoke-absorbing electrotome with an integrated telescopic smoke-absorbing pipe and an electrode protection sleeve thereof.

### Summary

The main objective of the utility model is to provide a smoke-absorbing electrotome with an integrated telescopic smoke-absorbing pipe and an electrode protection sleeve thereof, so as to solve the above technical problems.

In order to achieve the above objective, the utility model provides the following technical solution: Provided are a smoke-absorbing electrotome with an integrated telescopic smoke-absorbing pipe and an electrode protection sleeve thereof, including an electrotome electrode, a conductive sheet integrated electrode socket, a telescopic pipe, an electrotome handle and a rotating tail joint, the telescopic pipe being formed by integrated injection molding, a hexagonal angle lock being formed in an inner cavity of the telescopic pipe by integrated injection molding, the electrotome electrode and the conductive sheet integrated electrode socket being detachably connected to the hexagonal angle lock respectively, a rotating damping telescopic pipe locking mechanism being arranged between the telescopic pipe and the electrotome handle, and a smoke-discharging corrugated pipe being arranged at a rear end of the rotating tail joint; and
also including a multifunctional electrode protection sleeve, the multifunctional electrode protection sleeve being provided with a cleaning end, an electrode protection structure and a pulling end, and the multifunctional electrode protection sleeve being inserted in the telescopic pipe.

On the basis of the above solution and as a preferred solution of the above solution, the hexagonal angle lock includes a mounting block formed by integrated injection molding, the mounting block is provided with a mounting cavity, a hexagonal lock hole is arranged at a front end of the mounting cavity, and a circular hole is arranged at a rear end of the mounting cavity; a hexagonal lock block is arranged at a tail end of the electrotome electrode, and the hexagonal lock block is adapted to the hexagonal lock hole; and a front end of the conductive sheet integrated electrode socket is inserted in the circular hole.

On the basis of the above solution and as a preferred solution of the above solution, the rotating damping telescopic pipe locking mechanism includes a locking rotating cap, a damping ring is arranged in the locking rotating cap, and a rear end of the telescopic pipe is detachably connected to the damping ring.

On the basis of the above solution and as a preferred solution of the above solution, the damping ring is made of a rubber material.

On the basis of the above solution and as a preferred solution of the above solution, the electrotome handle includes a handle upper shell and a handle lower shell, and a printed circuit board (PCB) circuit component is mounted in the handle lower shell; the PCB circuit component is externally connected to a conductive wire, and a rear end of the conductive sheet integrated electrode socket is electrically connected to the PCB circuit component; and a control button is mounted at a top of the handle upper shell.

On the basis of the above solution and as a preferred solution of the above solution, a structure of a control button can be selectively set; and a top of the control button is a cap-shaped structure, a touch rod is arranged in a middle position of a bottom surface of the control button, and a bottom end of the touch rod is an inverted structure.

On the basis of the above solution and as a preferred solution of the above solution, the cleaning end of the multifunctional electrode protection sleeve includes a first body and a second body, bar-shaped arc grooves are molded on both the first body and the second body, a length of the first body is greater than a length of the second body, and a width of the first body is less than a width of the second body.

On the basis of the above solution and as a preferred solution of the above solution, the electrode protection structure includes a protection groove, a bottom wall of the protection groove is provided with a penetrated strip-shaped notch, and support blocks are arranged on both sides of one end of the strip-shaped notch.

On the basis of the above solution and as a preferred solution of the above solution, the pulling end includes a pulling head, a pulling hole is arranged in the pulling head, and the pulling hole is adapted to a head end of the electrotome electrode.

On the basis of the above solution and as a preferred solution of the above solution, the multifunctional electrode protection sleeve is formed by integrated injection molding, and the multifunctional electrode protection sleeve is made of an X-ray developing engineering plastic material.

The utility model has the following beneficial effects:
1. In the utility model, the telescopic pipe and the electrotome handle are both formed by integrated injection molding, thereby reducing a volume while avoiding a falling risk of a split smoke-absorbing pipe.
2. The electrotome handle and the telescopic pipe are locked or released by the rotating damping telescopic pipe locking mechanism to achieve an objective of expansion or contraction, and the rotating damping telescopic pipe locking mechanism is fixed at the front end of the handle lower shell. By an arrangement of the damping ring, a locking force is greatly increased, the locking of the telescopic pipe in any use state can be provided, excellent locking hand feeling can be provided, and the grease attached to a wall of the telescopic pipe during a surgery can also be scraped off. The utility model is applicable to different surgical needs.
3. The PCB circuit component is mounted on the handle lower shell, and the PCB circuit component is completely separated from a smoke-absorbing channel, thereby avoiding a damage to the circuit component from blood and body fluids during smoke absorption in a surgery.
4. The rotating tail joint is connected to the tail end of the electrotome handle and can rotate flexibly 180 degrees, thereby reducing obstruction of the smoke-discharging corrugated pipe.
5. By arranging the cap-shaped control button and forming the inverted structure at the bottom end of the touch rod of the control button, a better protection effect is achieved, and a risk of a button body being stuck by foreign matters such as blood clots is reduced.
6. During packaging, the cleaning end of the multifunctional electrode protection sleeve is inserted in the smoke-absorbing pipe, and the electrode protection structure is in contact with the electrotome electrode, thereby protecting the head of the electrotome electrode during transportation of a high-frequency electrotome, and preventing a damage to a packaging bag caused by the electrotome electrode and a damage to the electrotome electrode caused by an external force. During a surgery, human tissues may cause blockage of the smoke-absorbing pipe, and thus, the cleaning end of the multifunctional electrode protection sleeve can be used for unblocking. During replacement of the electrotome electrode, the pulling head on the pulling end of the multifunctional electrode protection sleeve can be used for effectively disassembling and assembling the electrotome electrode.

### Brief Description of Figures

FIG. 1 is a schematic view of an overall structure of the utility model;
FIG. 2 is an exploded view of the utility model;
FIG. 3 is a partial sectional view of a locking rotating cap and a damping ring in the utility model;
FIG. 4 is a sectional view of a control button in the utility model;
FIG. 5 is an assembly diagram of a multifunctional electrode protection sleeve and a conductive sheet integrated electrode socket in the utility model;
FIG. 6 is a schematic view of an overall structure of a multifunctional electrode protection sleeve in the utility model;
FIG. 7 is a top view of a multifunctional electrode protection sleeve in the utility model;
FIG. 8 is a schematic view of an assembly structure of a multifunctional electrode protection sleeve and a smoke-absorbing electrotome in the utility model;
FIG. 9 is an exploded view of a multifunctional electrode protection sleeve and a smoke-absorbing electrotome in the utility model; and
FIG. 10 is a front view of the utility model.

In figures: 1-telescopic pipe; 10-multifunctional electrode protection sleeve; 11-cleaning end; 110-first body; 111-second body; 1100-bar-shaped arc groove; 12-electrode protection structure; 120-protection groove; 121-strip-shaped notch; 122-support block; 13-pulling end; 130-pulling hole; 131-pulling head; 14-mounting block; 140-hexagonal lock hole; 2-electrotome handle; 3-electrotome electrode; 4-conductive sheet integrated electrode socket; 5-PCB circuit component; 6-handle upper shell; 7-control button; 70-cap-shaped structure; 71-inverted structure; 72-button body; 73-elastic arm; 8-rotating tail joint; 9-locking rotating cap; and 90-damping ring.

### Detailed Description

To make the objectives, technical solutions and advantages of the present application clearer, the technical solutions in embodiments are clearly and completely described below with reference to accompanying drawings in the embodiments. However, the specific implementations and embodiments described below are for illustrative purposes only, and are not intended to limit the utility model.

In the description of the utility model, it should be understood that the orientation or position relationships indicated by the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. are based on the orientation or position relationships shown in the accompanying drawings, and are intended to facilitate the description of the utility model and simplify the description only, rather than indicating or implying that the apparatus or element referred to must have a particular orientation or be constructed and operated in a particular orientation, and therefore are not to be interpreted as limitations on the utility model.

In the utility model, unless otherwise clearly specified and defined, the terms "mount", "connected", "connect", "fix", etc. should be broadly understood. For example, the connection may be fixed connection, detachable connection or integrated connection, may be mechanical connection or electrical connection, or may be direct connection, indirect connection through an intermediate, internal communication between two elements or interaction relationship between two elements unless otherwise clearly specified. For those of ordinary skill in the art, the specific meanings of the above terms in the utility model can be understood according to specific situations.

In the utility model, unless otherwise clearly specified and defined, the first feature "above" or "below" the second feature may be direct contact between the first feature and the second feature, or indirect contact between the first feature and the second feature through an intermediate. Moreover, the first feature "over", "above" and "on" the second feature may mean that the first feature is directly above or obliquely above the second feature, or may only indicate that the level of the first feature is higher than that of the second feature. The first feature "under", "below" and "underneath" the second feature may mean that the first feature is directly below or obliquely below the second feature, or may only indicate that the level of the first feature is less than that of the second feature.

### Embodiment I

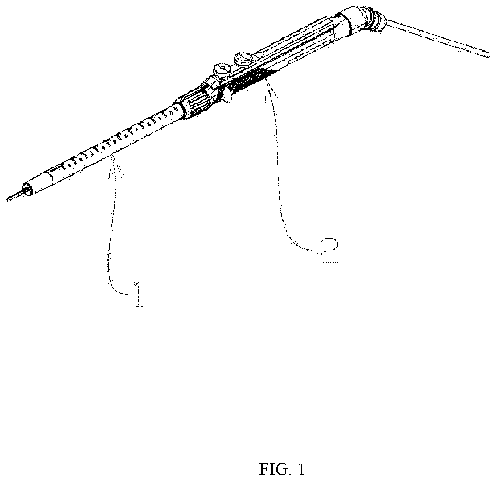

Referring to FIG. 1 to FIG. 2 in the accompanying drawings, this embodiment provides a smoke-absorbing electrotome with an integrated telescopic smoke-absorbing pipe and an electrode protection sleeve thereof, including an electrotome electrode 3, a conductive sheet integrated electrode socket 4, a telescopic pipe 1, an electrotome handle 2 and a rotating tail joint 8. The telescopic pipe 1 is formed by integrated injection molding. A hexagonal angle lock is formed in an inner cavity of the telescopic pipe 1 by integrated injection molding. The electrotome electrode 3 and the conductive sheet integrated electrode socket 4 are detachably connected to the hexagonal angle lock respectively. A rotating damping telescopic pipe locking mechanism is arranged between the telescopic pipe 1 and the electrotome handle 2. A smoke-discharging corrugated pipe is arranged at a rear end of the rotating tail joint 8.

The integrated telescopic smoke-absorbing pipe and an electrode protection sleeve thereof also include a multifunctional electrode protection sleeve 10. The multifunctional electrode protection sleeve 10 is provided with a cleaning end 11, an electrode protection structure 12 and a pulling end 13, and the multifunctional electrode protection sleeve 10 is inserted in the telescopic pipe 1.

In this embodiment, the telescopic pipe 1 and the electrotome handle 2 are both formed by integrated injection molding, thereby reducing the volume while avoiding the falling risk of a split smoke-absorbing pipe.

Further, the electrotome handle includes a handle upper shell and a handle lower shell, and a PCB circuit component 5 is mounted in the handle lower shell; the PCB circuit component 5 is externally connected to a conductive wire, and a rear end of the conductive sheet integrated electrode socket 4 is electrically connected to the PCB circuit component 5; and a control button 7 is mounted at a top of the handle upper shell 6.

Specifically, a mounting groove 20 is integrally molded at a top of the handle lower shell, and the PCB circuit component 5 is arranged in the mounting groove 20; the handle upper shell 6 is arranged at a top of the mounting groove 20, and the control button is mounted on a top surface of the handle upper shell 6; and the PCB circuit component 5 is mounted on the mounting groove 20 of the electrotome handle 2, and the PCB circuit component 5 is completely separated from a smoke-absorbing channel, thereby avoiding the damage to the circuit component from blood and body fluids during smoke absorption in a surgery.

Furthermore, the smoke-absorbing channel is arranged in the electrotome handle 2, and the smoke-absorbing channel is arranged below the mounting groove 20.

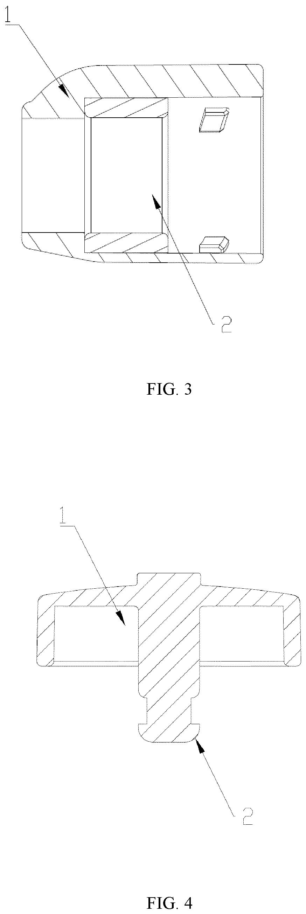

More specifically, referring to FIG. 4 in the accompanying drawings, the structure of the control button 7 can be selectively set; and in this embodiment, a top of the control button 7 is a cap-shaped structure 70, a touch rod is arranged in a middle position of a bottom surface of the control button 7, and a bottom end of the touch rod is an inverted structure 71.

By arranging the cap-shaped control button 7 and forming the inverted structure 71 at the bottom end of the touch rod of the control button 7, a better protection effect is achieved, and the risk of the control button being stuck by foreign matters such as blood clots is reduced.

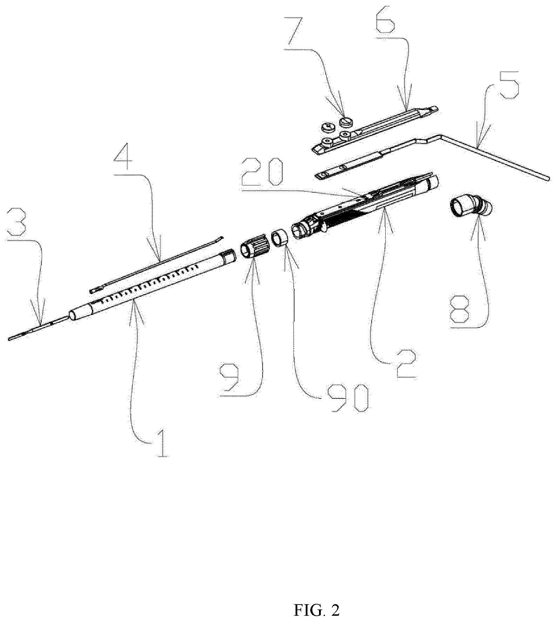

Further, referring to FIG. 2 and FIG. 3 in the accompanying drawings, the rotating damping telescopic pipe locking mechanism includes a locking rotating cap 9, a damping ring 90 is arranged in the locking rotating cap 9, and a rear end of the telescopic pipe 4 is detachably connected to the damping ring 90.

Preferably, the damping ring 90 is made of a rubber material.

The electrotome handle 2 and the telescopic pipe 1 are locked or released by the rotating damping telescopic pipe locking mechanism to achieve the objective of expansion or contraction, and the rotating damping telescopic pipe locking mechanism is fixed at a front end of the handle lower shell. By the arrangement of the damping ring 90, the locking force is greatly increased, the locking of the telescopic pipe 1 in any use state can be provided, excellent locking hand feeling can be provided, and the grease attached to the wall of the telescopic pipe 1 during a surgery can also be scraped off. The utility model is applicable to different surgical needs.

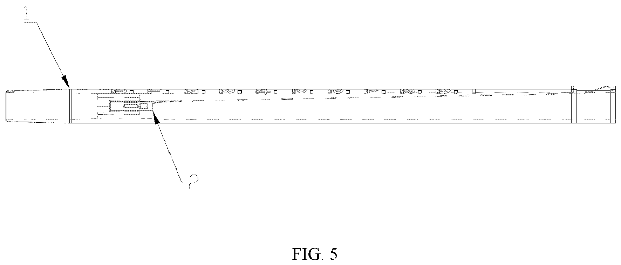

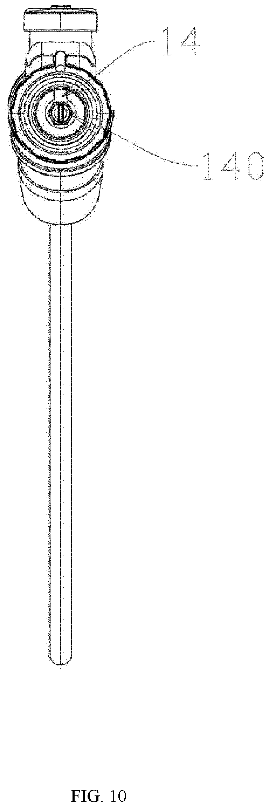

Further, referring to FIG. 5 and FIG. 10 in the accompanying drawings, the hexagonal angle lock includes a mounting block 14 formed by integrated injection molding, the mounting block 14 is provided with a mounting cavity, a hexagonal lock hole 140 is arranged at a front end of the mounting cavity, and a circular hole is arranged at a rear end of the mounting cavity; a hexagonal lock block is arranged at a tail end of the electrotome electrode 3, and the hexagonal lock block is adapted to the hexagonal lock hole 140; and a front end of the conductive sheet integrated electrode socket 4 is inserted in the circular hole.

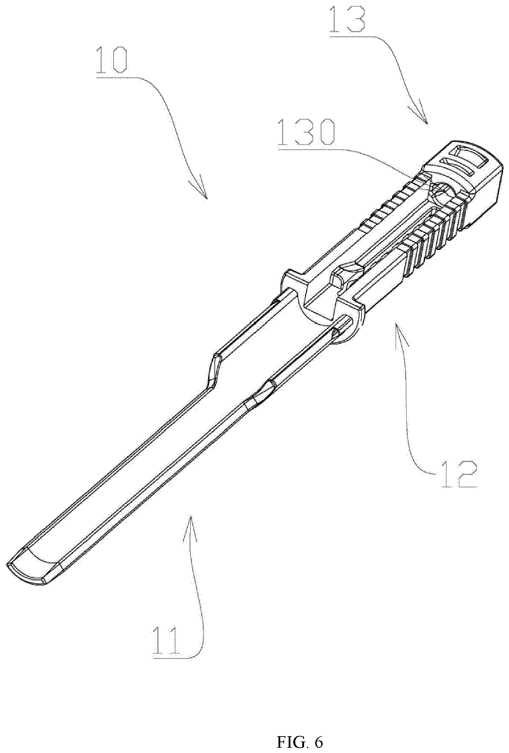

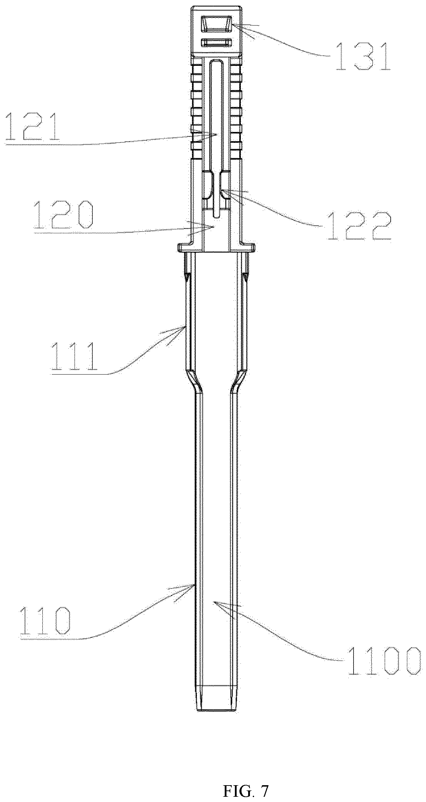

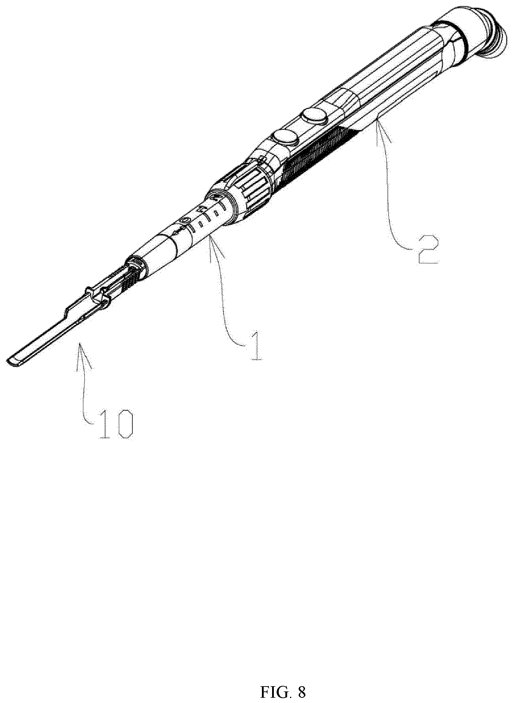

Referring to FIG. 6 to FIG. 8 in the accompanying drawings, in this embodiment, the cleaning end 11 of the multifunctional electrode protection sleeve 10 includes a first body 110 and a second body 111, bar-shaped arc grooves 1100 are molded on both the first body 110 and the second body 111, the length of the first body 110 is greater than the length of the second body 111, and the width of the first body 110 is less than the width of the second body 111.

Further, the electrode protection structure 12 includes a protection groove 120, a bottom wall of the protection groove 120 is provided with a penetrated strip-shaped notch 121, and support blocks 122 are arranged on both sides of one end of the strip-shaped notch 121.

Further, the pulling end 13 includes a pulling head 131, a pulling hole 130 is arranged in the pulling head 131, and the pulling hole 130 is adapted to a head end of the electrotome electrode 3.

Preferably, the multifunctional electrode protection sleeve 10 is formed by integrated injection molding, and the multifunctional electrode protection sleeve is made of an X-ray developing engineering plastic material.

A use method of the multifunctional electrode protection sleeve 10 is as follows: During packaging, the cleaning end 11 of the multifunctional electrode protection sleeve 10 is inserted in the smoke-absorbing pipe 1, and the electrode protection structure 12 is in contact with the electrotome electrode 3, thereby protecting the head of the electrotome electrode during transportation of a high-frequency electrotome, and preventing the damage to a packaging bag caused by the electrotome electrode 3 and the damage to the electrotome electrode caused by an external force. During a surgery, human tissues may cause blockage of the smoke-absorbing pipe, and thus, the cleaning end 11 of the multifunctional electrode protection sleeve 10 can be used for unblocking. During replacement of the electrotome electrode 3, the pulling head on the pulling end of the multifunctional electrode protection sleeve 10 can be used for effectively disassembling and assembling the electrotome electrode 3.

### Embodiment II

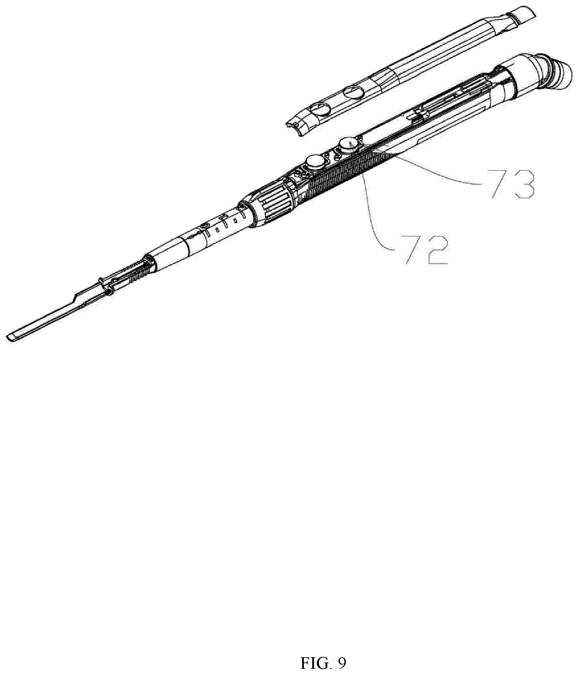

Referring to FIG. 9 in the accompanying drawings, different from Embodiment I, a control button in this embodiment includes a button body 72, elastic arms 73 are arranged on both sides of the button body 72, and a thickness of the button body 72 is thinner than that of the button body 72 in Embodiment I. To increase the user experience, the button body 72 is effectively positioned by the design of the elastic arms 73 on the button body 72. The positioning is excellent, a better button function is achieved, and the impact of body fluids or surgical fluids on the function of the button body can also be better prevented.

The above embodiments are only preferred embodiments of the utility model, and are not intended to limit the scope of protection of the utility model. Therefore, all equivalent changes made according to the structure, shape and principle of the utility model should be covered within the scope of protection of the utility model.

## Claims

1. A smoke-absorbing electrotome with an integrated telescopic smoke-absorbing pipe and an electrode protection sleeve thereof, **characterized by** comprising an electrode, a conductive sheet integrated electrode socket, a telescopic pipe, an electrotome handle and a rotating tail joint, the telescopic pipe being formed by integrated injection molding, a hexagonal angle lock being formed in an inner cavity of the telescopic pipe by integrated injection molding, the electrotome electrode and the conductive sheet integrated electrode socket being detachably connected to the hexagonal angle lock respectively, a rotating damping telescopic pipe locking mechanism being arranged between the telescopic pipe and the electrotome handle, and a smoke-discharging corrugated pipe being arranged at a rear end of the rotating tail joint; and
also comprising a multifunctional electrode protection sleeve, the multifunctional electrode protection sleeve being provided with a cleaning end, an electrode protection structure and a pulling end, and the multifunctional electrode protection sleeve being inserted in the telescopic pipe.

2. The smoke-absorbing electrotome with an integrated telescopic smoke-absorbing pipe and the electrode protection sleeve thereof according to claim 1, **characterized in that** the hexagonal angle lock comprises a mounting block formed by integrated injection molding, the mounting block is provided with a mounting cavity, a hexagonal lock hole is arranged at a front end of the mounting cavity, and a circular hole is arranged at a rear end of the mounting cavity; a hexagonal lock block is arranged at a tail end of the electrotome electrode, and the hexagonal lock block is adapted to the hexagonal lock hole; and a front end of the conductive sheet integrated electrode socket is inserted in the circular hole.

3. The smoke-absorbing electrotome with an integrated telescopic smoke-absorbing pipe and the electrode protection sleeve thereof according to claim 1, **characterized in that** the rotating damping telescopic pipe locking mechanism comprises a locking rotating cap, a damping ring is arranged in the locking rotating cap, and a rear end of the telescopic pipe is detachably connected to the damping ring.

4. The smoke-absorbing electrotome with an integrated telescopic smoke-absorbing pipe and the electrode protection sleeve thereof according to claim 3, **characterized in that** the damping ring is made of a rubber material.

5. The smoke-absorbing electrotome with an integrated telescopic smoke-absorbing pipe and the electrode protection sleeve thereof according to claim 1, **characterized in that** the electrotome handle comprises a handle upper shell and a handle lower shell, and a printed circuit board (PCB) circuit component is mounted in the handle lower shell; the PCB circuit component is externally connected to a conductive wire, and a rear end of the conductive sheet integrated electrode socket is electrically connected to the PCB circuit component; and a control button is mounted at a top of the handle upper shell.

6. The smoke-absorbing electrotome with an integrated telescopic smoke-absorbing pipe and the electrode protection sleeve thereof according to claim 1, **characterized in that** a structure of a control button can be selectively set; and a top of the control button is a cap-shaped structure, a touch rod is arranged in a middle position of a bottom surface of the control button, and a bottom end of the touch rod is an inverted structure.

7. The smoke-absorbing electrotome with an integrated telescopic smoke-absorbing pipe and the electrode protection sleeve thereof according to claim 1, **characterized in that** the cleaning end of the multifunctional electrode protection sleeve comprises a first body and a second body, bar-shaped arc grooves are molded on both the first body and the second body, a length of the first body is greater than a length of the second body, and a width of the first body is less than a width of the second body.

8. The smoke-absorbing electrotome with an integrated telescopic smoke-absorbing pipe and the electrode protection sleeve thereof according to claim 7, **characterized in that** the electrode protection structure comprises a protection groove, a bottom wall of the protection groove is provided with a penetrated strip-shaped notch, and support blocks are arranged on both sides of one end of the strip-shaped notch.

9. The smoke-absorbing electrotome with an integrated telescopic smoke-absorbing pipe and the electrode protection sleeve thereof according to claim 8, **characterized in that** the pulling end comprises a pulling head, a pulling hole is arranged in the pulling head, and the pulling hole is adapted to a head end of the electrotome electrode.

10. The smoke-absorbing electrotome with an integrated telescopic smoke-absorbing pipe and the electrode protection sleeve thereof according to claim 1, **characterized in that** the multifunctional electrode protection sleeve is formed by integrated injection molding, and the multifunctional electrode protection sleeve is made of an X-ray developing engineering plastic material.
